# EUROPEAN PATENT APPLICATION

(11) **EP 2 510 902 A1**
(43) Date of publication of application: **17.10.2012**
(21) Application number: 11162721.2
(22) Date of filing: 15.04.2011
(51) Int. Cl.: A61F 2/00, A61M 5/42

(54) **Intraocular injection device**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is a device for intraocular injection comprising a drug delivery device, a foot (5) at a distal end of the drug delivery device for positioning the drug delivery device with respect to an injection site, and a magnifying device (8) coupled to one of the drug delivery device and the foot for magnifying a view of the injection site.

## Description

The present invention relates to a drug delivery device, especially an apparatus for an intraocular injection.

### Background

In the field of intraocular injections, it is generally important to visualize the injection site prior to, during and after the injection. However, given the small operating space, such visualization is difficult. US 4,743,234 and US 6,001,082 disclose magnifier means for use with hypodermic syringes. However, the known magnifying means do not improve the visibility of the injection site but rather hinder a direct view of the injection site and are cumbersome in use.

### Summary of the Invention

In an exemplary embodiment, the present invention comprises a device for intraocular injection comprising a drug delivery device, a foot at a distal end of the drug delivery device for positioning the drug delivery device with respect to an injection site, and a magnifying device coupled to one of the drug delivery device and the foot for magnifying a view of the injection site. The drug delivery device comprises a housing compartment for receiving a cartridge or a syringe. The drug delivery device comprises a spring biasing a needle arrangement of the cartridge in a proximal position.

The magnifying device may comprise a prism element, preferably an annular prism element, having its focus on the injection site. The magnifying device may be located at a distal end of the drug delivery device or a proximal end of the foot. The magnifying device may comprise a barrel lens located at a distal end of the drug delivery device or on the foot. The magnifying device may comprise a lens located at a distal end of the foot.

The magnifying device may be an integral part of the drug delivery device or permanently attached to the drug delivery device. In another exemplary embodiment, the magnifying device may be releasably attached to the drug delivery device or the foot.

The foot may include a ring for aligning with the cornea.

### Brief Description of the Drawings

In the following, the invention will be described by way of an example and with reference to the schematic drawings in which:
Fig. 1 shows a sectional view of a distal part of a device for intraocular injection according to an exemplary embodiment of the present invention;
Fig. 2 shows a sectional view of the distal part of the exemplary embodiment of the device for intraocular injection shown in Fig. 1,
Fig. 3 shows an isometric view of the distal part of the exemplary embodiment of the device for intraocular injection shown in Fig. 1,
Fig. 4 shows a sectional view of a foot similar to the distal portion of the exemplary embodiment of the device for intraocular injection shown in Fig. 1,
Fig. 5 shows an isometric view of the exemplary embodiment of the foot shown in Fig. 4,
Fig. 6 shows a sectional view of the distal part of a device for intraocular injection according to an exemplary embodiment of the present invention,
Fig. 7 shows a sectional view of the distal part of the exemplary embodiment of the device for intraocular injection shown in Fig. 6,
Fig. 8 shows an isometric view of the distal part of the exemplary embodiment of the device for intraocular injection shown in Fig. 6,
Fig. 9 shows an isometric view of a foot similar to the distal part of the exemplary embodiment of the device for intraocular injection shown in Fig. 6,
Fig. 10 shows a sectional view of the exemplary embodiment of the foot shown in Fig. 9,
Fig. 11 shows a sectional view of the distal part of a device for intraocular injection according to an exemplary embodiment of the present invention, and
Fig. 12 shows a sectional view of the distal part of the exemplary embodiment of the device for intraocular injection shown in Fig. 11.

### Detailed Description

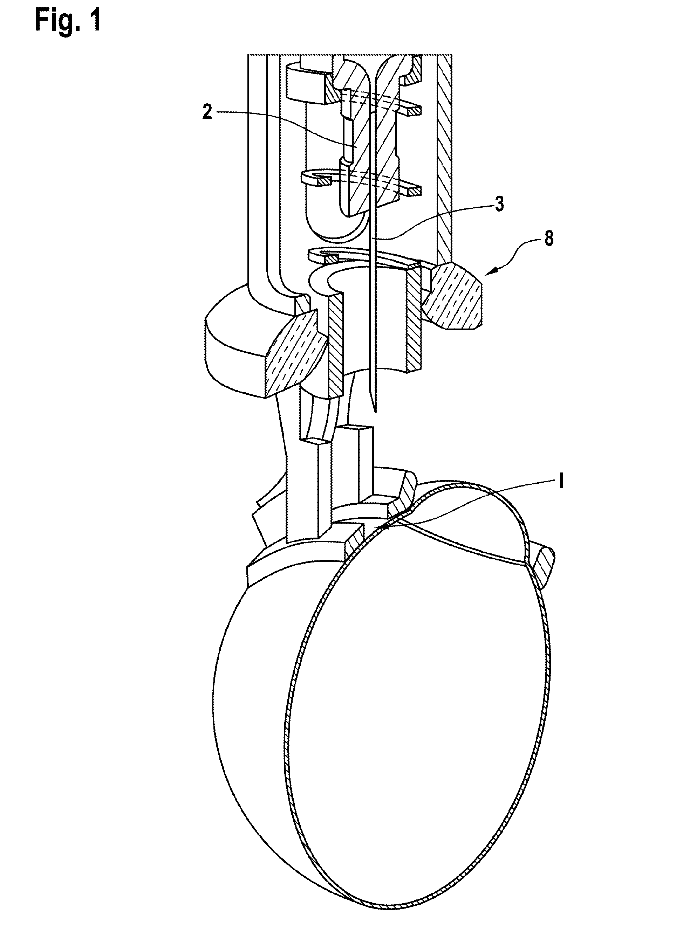

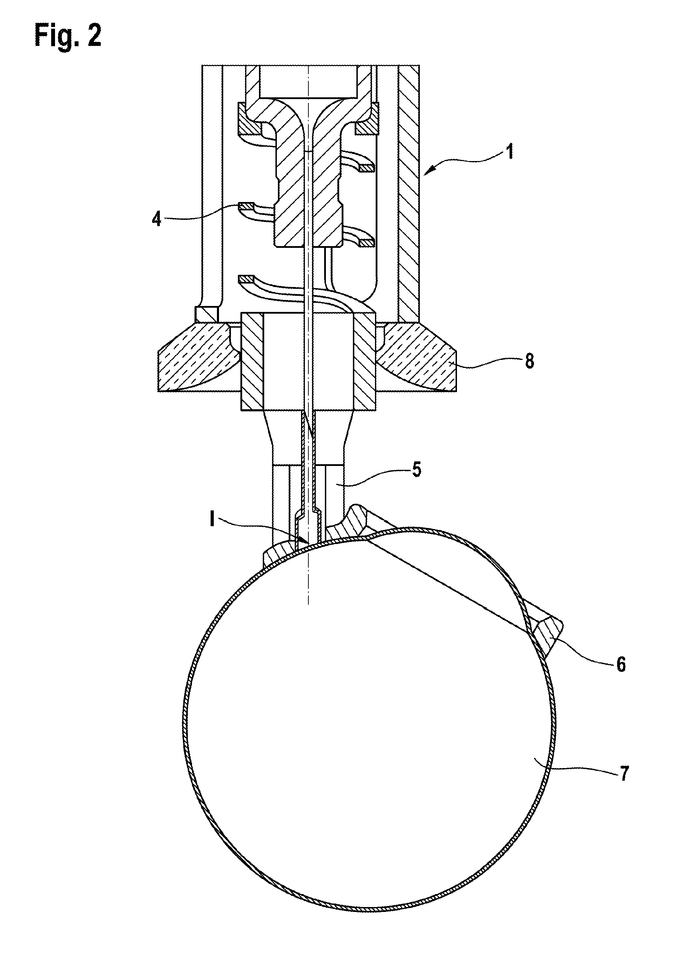

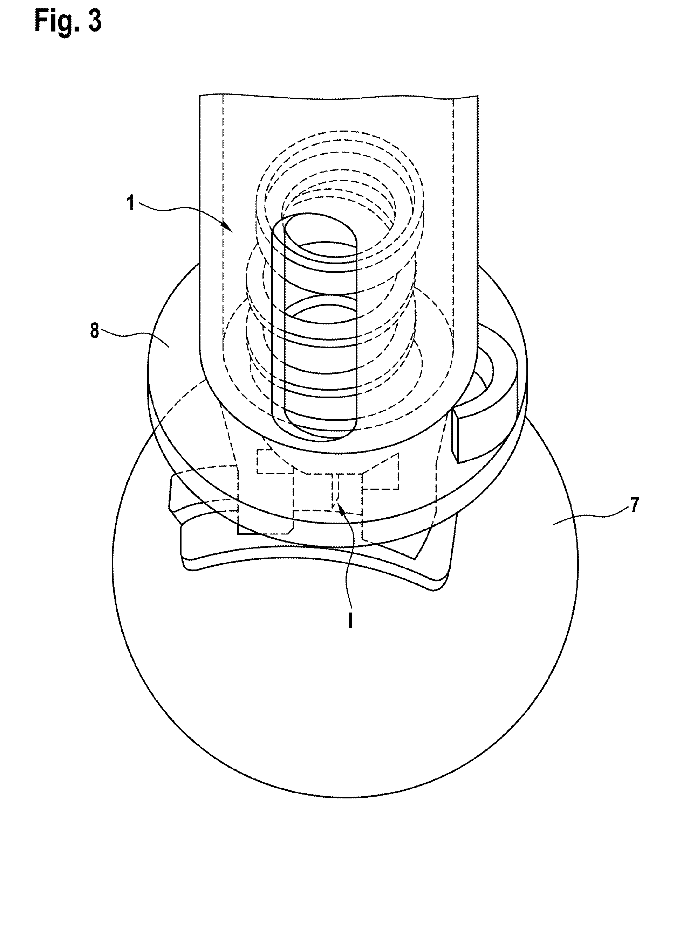

Figures 1 to 3 show an exemplary embodiment of a device for intraocular injection 1, more particularly an intraocular injection device. A body of the device 1 may form a housing compartment for receiving a cartridge 2 (or a drug delivery device, e.g., a syringe) containing a medicinal product, for example steroids or monoclonal antibodies used to treat macular degeneration. A distal end (lower end in the figures) of the cartridge 2 is provided with a needle arrangement 3. Those of skill in the art will understand that the combination of the cartridge 2 and the needle arrangement 3 may be in the form of a prefilled syringe. The drug delivery device may further comprise a spring 4 arranged within the housing compartment to bias the cartridge 2 together with the needle arrangement 3 in the proximal direction. The cartridge 2 together with the needle arrangement 3 may be pushed in the distal direction during injection thus compressing the spring 4 (until it abuts the distal end of the housing compartment) and administering the medicinal product.

In an exemplary embodiment, the device 1 may include a foot 5 or spacer located at its distal end. The foot 5 may be provided with a positioning device to aid the accurate placement of the device relative to an injection site I. In an exemplary embodiment shown in Figures 1 to 3, the foot 5 is provided with a ring 6 which may be sized and shaped to encircle a cornea of a human eye 7. In use, the foot 5 is placed in contact with the eye 7 and the ring 6 is positioned around the cornea. Those of skill in the art will understand that the positioning device may be any size or shape (e.g., circular, semi-circular, arcuate, etc.) which facilitates placement and positioning of the drug delivery device 1. In an exemplary embodiment, the positioning device may have a width which ensures that the injection will not penetrate the cornea, but will be directed through the conjunctiva and sclera into the vitreous body.

In the exemplary embodiment shown in Figures 1 to 3, a magnifying device 8 is provided and positioned to magnify a view of the injection site I. In an exemplary embodiment, the magnifying device 8 may be an annular prism lens formed around a circumference of a distal portion of the drug delivery device 1. In the exemplary embodiment, the use of an annular prism lens may facilitate viewing of the injection site I from multiple angles. However, those of skill in the art will understand that the magnifying device 8 may be any size and/or shape and be located at any position on or relative to the device 1, so long as there is a clear line of sight between the magnifying device 8 and the injection site I.

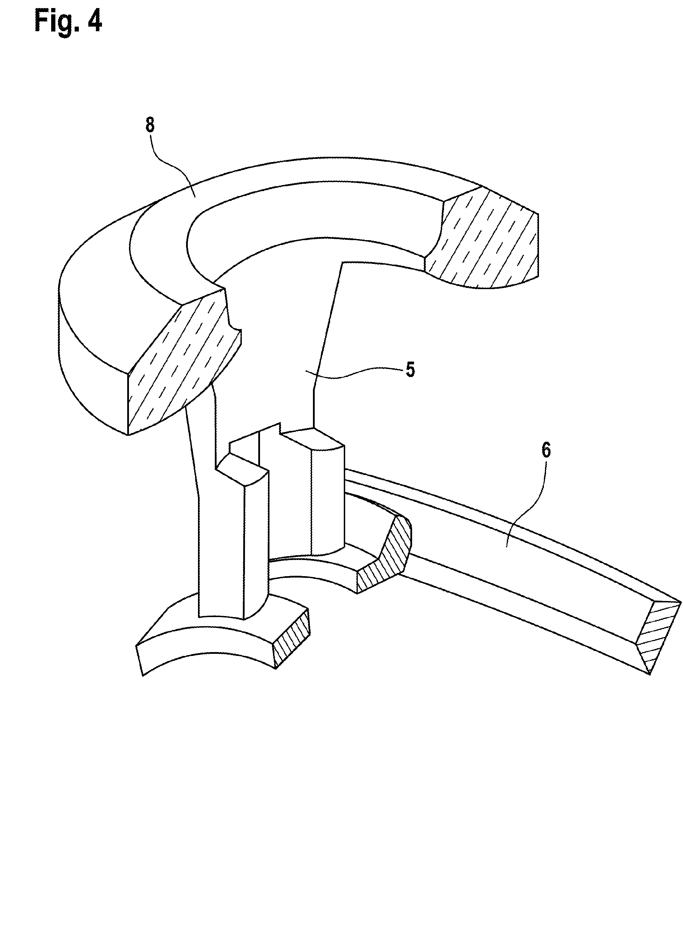

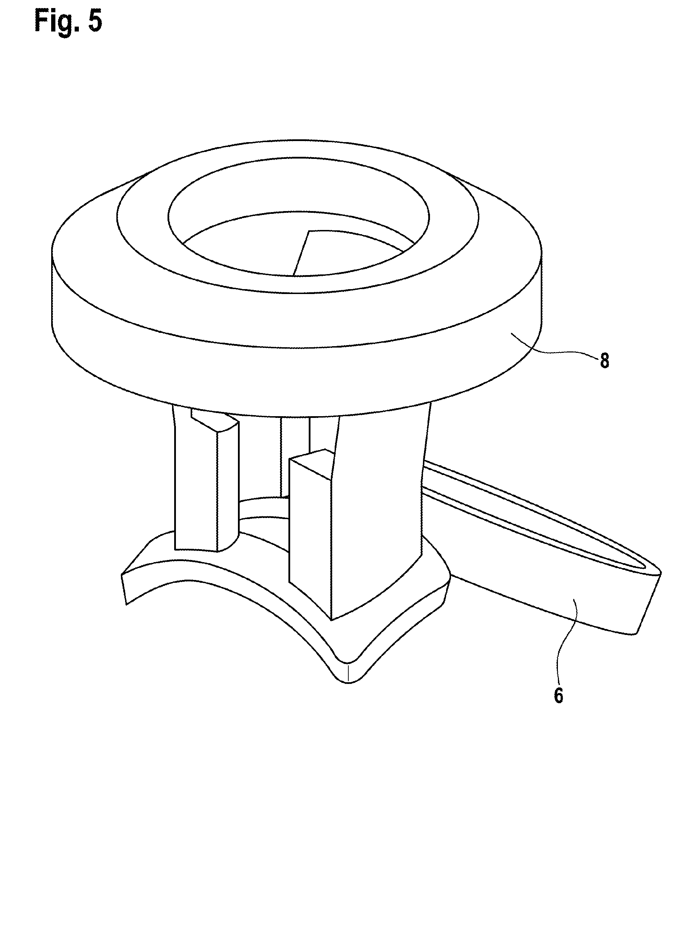

In an exemplary embodiment shown in Figures 4 and 5, the foot 5 is shown as a separate element which may be releasably or permanently attached to, or formed integrally with, the body of a device 1. In an exemplary embodiment in which the foot 5 is separable from the body of the device 1, a proximal portion of the foot 5 may include a coupling mechanism, e.g., threads, snap-fit, bayonet-fit, friction fit, etc., for coupling to a distal end of the body. The coupling mechanism may also include an audible feedback ("click") when it has been secured to the body of the device 1.

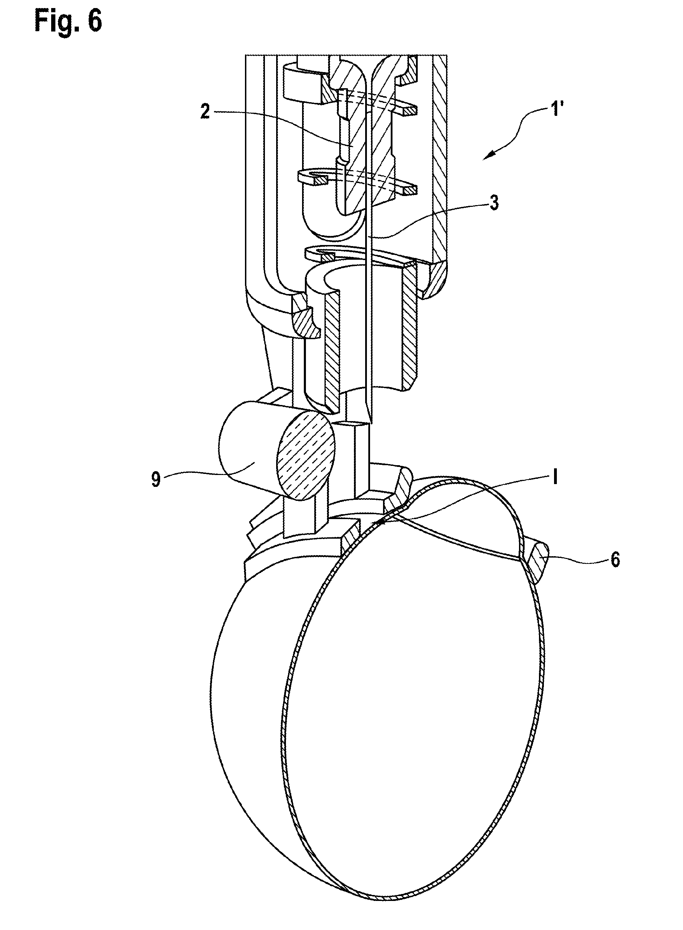

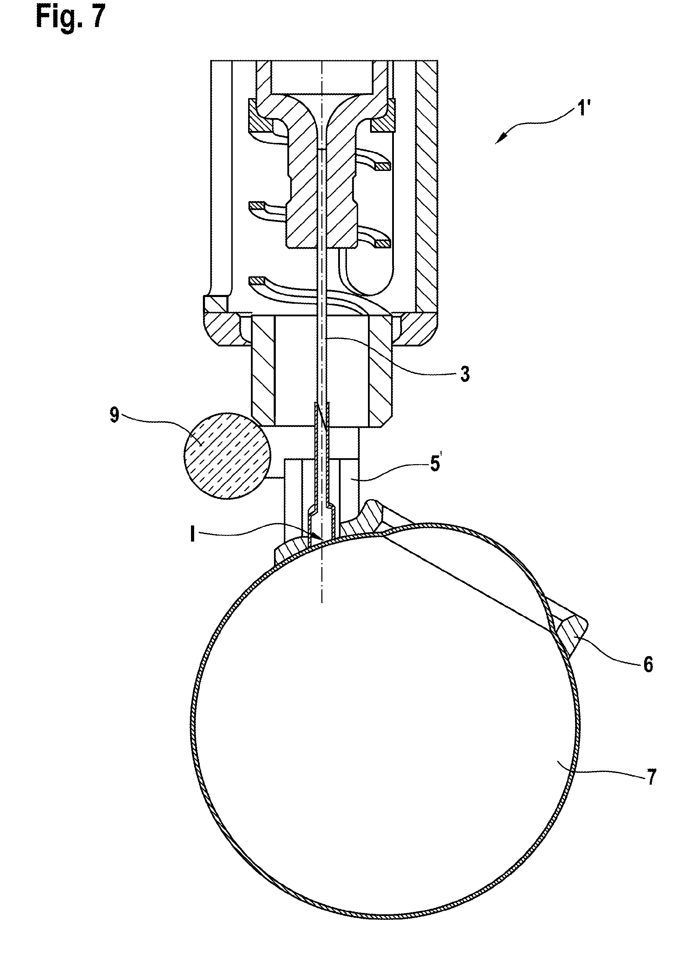

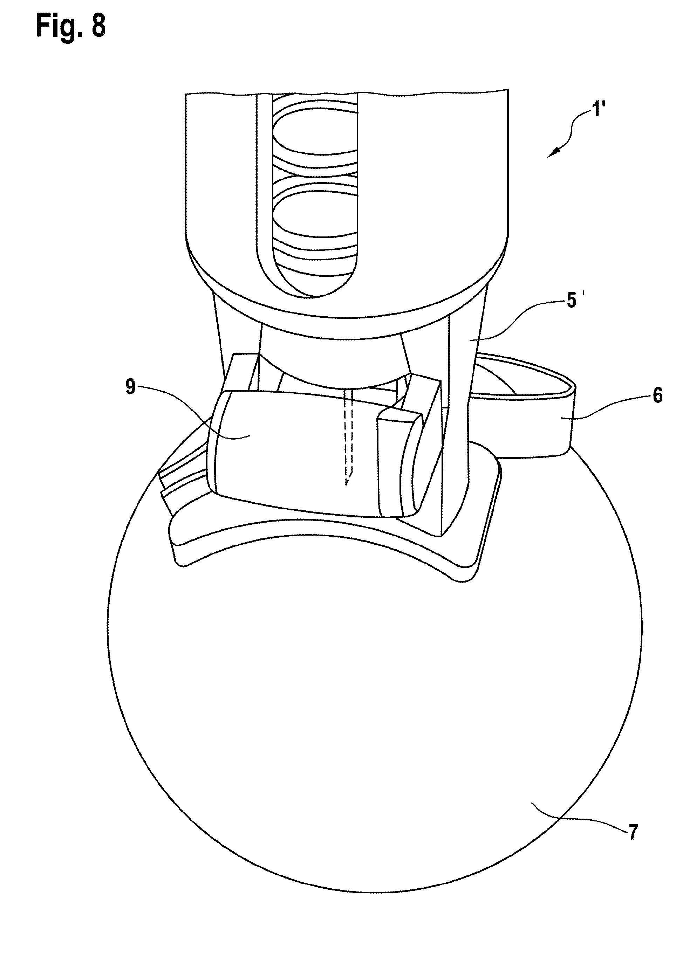

Figures 6 to 8 depict another exemplary embodiment of a device for intraocular injection 1'. In this exemplary embodiment, the magnifying device may be a barrel lens 9 provided on the foot 5' to provide a magnified view of the injection site I. The barrel lens 9 may be substantially designed as a cylindrical body having an axis extending perpendicular to an axis of the device 1'. In another exemplary embodiment, the magnifying device may be a disc-shaped lens provided on the foot 5'.

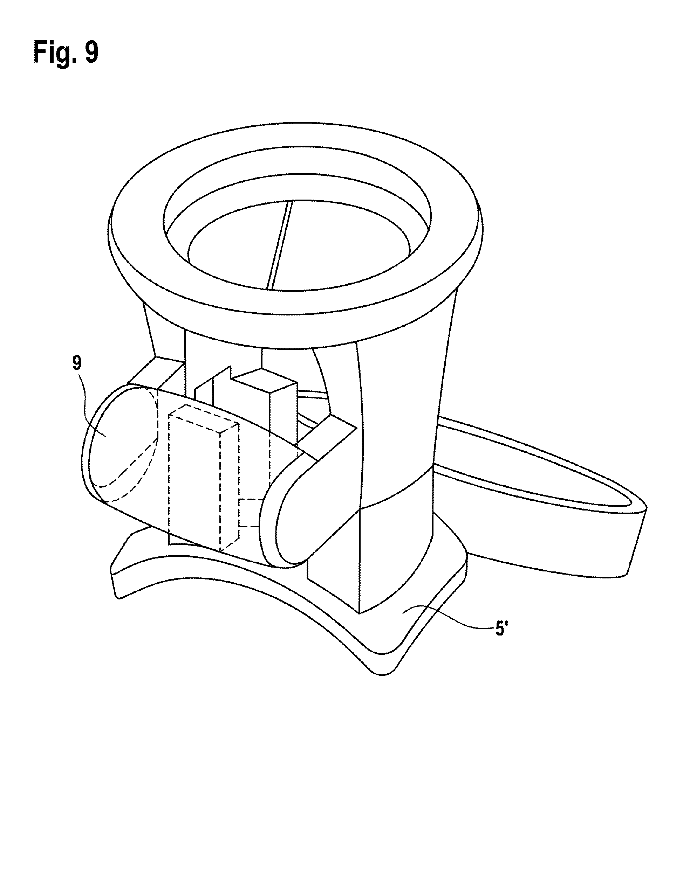

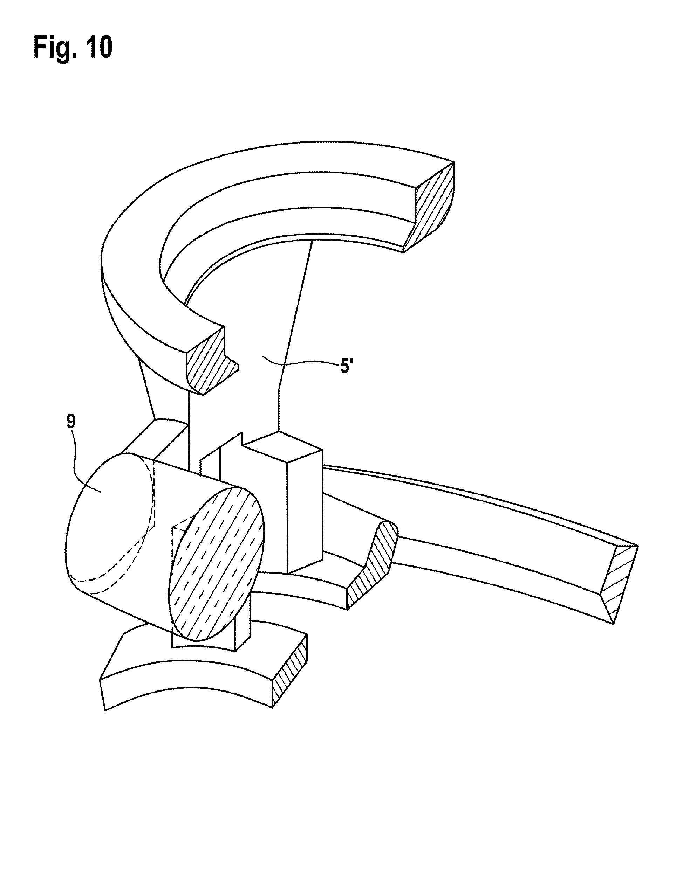

As shown in the exemplary embodiments depicted in Figures 9 and 10, the barrel lens 9 may be provided as an integral part of the foot 5'. Alternatively, the barrel lens 9 may be separable from the foot 5'. In this exemplary embodiment, the foot 5' may either be an integral part of the device 1' or may be provided as a separate part for attachment to the device 1', as described above.

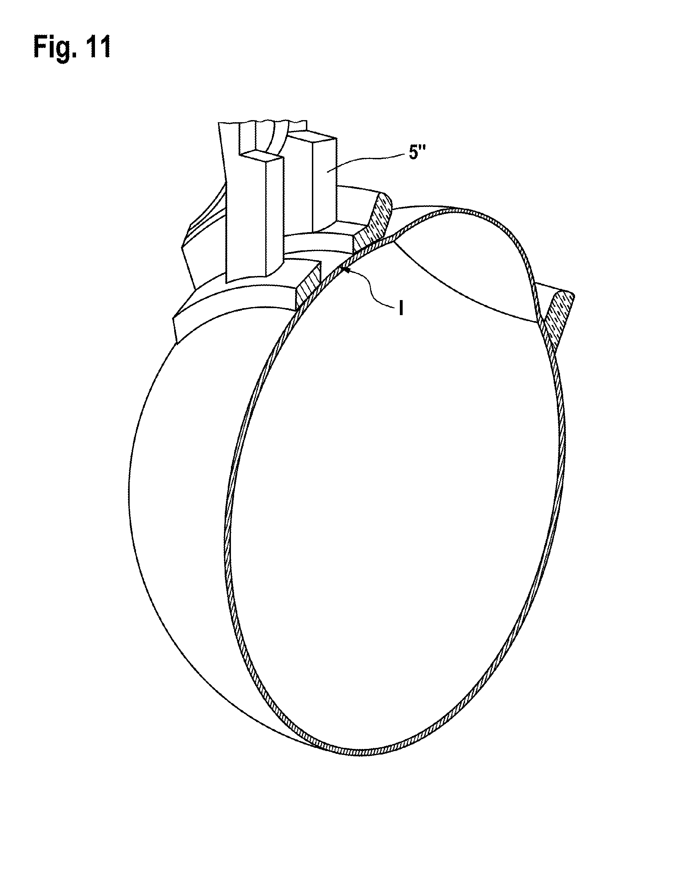

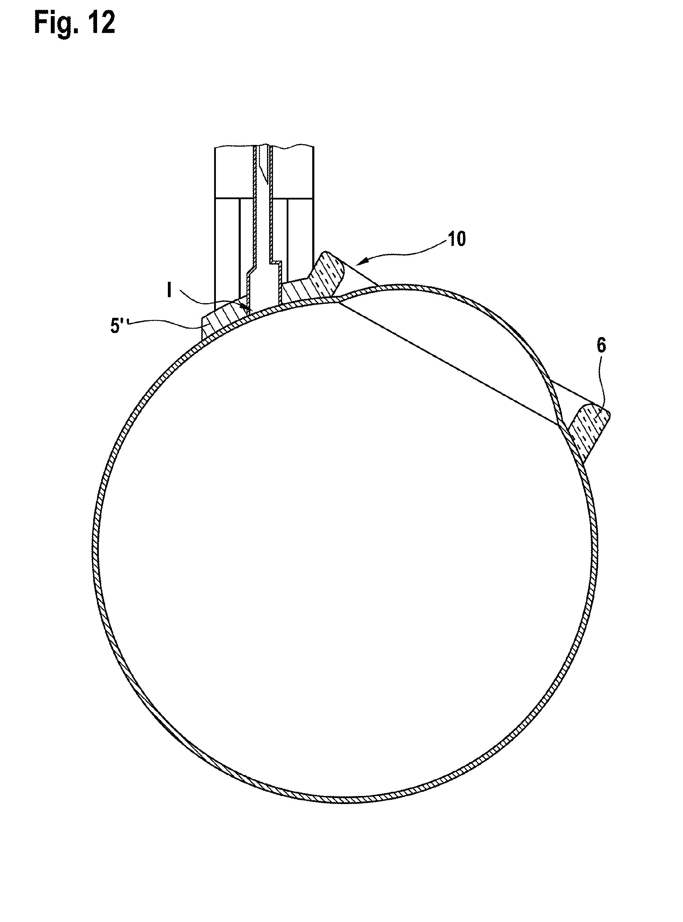

A further exemplary embodiment of the present invention is shown in Figures 11 and 12 depicting a distal portion of a device for intraocular injection including a foot 5" having a magnifying property. For example, a lens element 10 may be integrated into the foot 5", or the foot 5" may be manufactured from a material with a magnifying property.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A device for intraocular injection comprising:
a drug delivery device;
a foot (5) at a distal end of the drug delivery device for positioning the drug delivery device with respect to an injection site; and
a magnifying device (8) coupled to one of the drug delivery device and the foot (5) for magnifying a view of the injection site.

2. The device according to claim 1, wherein the drug delivery device comprises a housing compartment for receiving a cartridge (2) or a syringe.

3. The device according to claims 1 or 2, wherein the drug delivery device comprises a spring (4) biasing a needle arrangement (3) of the cartridge (2) in a proximal position.

4. The device according to any of the preceding claims, wherein the magnifying device (8) comprises a prism element, preferably an annular prism element, having its focus on the injection site.

5. The device according to any of the preceding claims, wherein the magnifying device (8) is located at a distal end of the drug delivery device or a proximal end of the foot (5).

6. The device according to claims 1 to 3, wherein the magnifying device (8) comprises a barrel lens (9).

7. The device according to claims 2 and 6, wherein the barrel lens (9) is located at a distal end of the drug delivery device or on the foot (5).

8. The device according to claims 1 to 3, wherein the magnifying device (8) comprises a lens (10) located at a distal end of the foot (5).

9. The device according to any of the preceding claims, wherein the magnifying device (8) is an integral part of the drug delivery device or permanently attached to the drug delivery device.

10. The device according to claims 1 to 8, wherein the magnifying device (8) is releasably attached to the drug delivery device or the foot (5).

11. The device according to any of the preceding claims, wherein the foot (5) includes a ring for aligning with the cornea.
